# EUROPÄISCHE PATENTSCHRIFT

(11) **EP 0 462 150 B1**
(45) Veröffentlichungstag und Bekanntmachung des Hinweises auf die Patenterteilung: **07.09.1994**
(21) Anmeldenummer: 90904270.7
(22) Anmeldetag: 08.03.1990
(51) Int. Cl.: C07D 333/32, C07C 217/36, A61K 31/38, A61K 31/13

(54) **NEUE ARYLOXY-ALKYLAMINE, DEREN HERSTELLUNG UND DIESE ENTHALTENDE ARZNEIMITTEL**
NOVEL ARYLOXY ALKYL AMINES, THEIR PRODUCTION AND MEDICAMENTS CONTAINING THEM
NOUVELLES ARYLOXY-ALKYLAMINES, LEUR FABRICATION ET MEDICAMENTS LES CONTENANT

(30) Priorität: 08.03.1989 DE 3907512
(43) Veröffentlichungstag der Anmeldung: 27.12.1991
(73) Patentinhaber: Laevosan-Gesellschaft m.b.H., A-4020 Linz (AT)
(72) Erfinder: LOTZ, Bernhard, A-4020 Linz (AT); GREIER, Gerhard, A-4020 Linz (AT)
(74) Vertreter: Weickmann, Heinrich, Dipl.-Ing.
(86) Internationale Anmeldenummer: EP9000380
(87) Internationale Veröffentlichungsnummer: WO9010628

(56) Entgegenhaltungen:
- EP-A- 362 707
- EP-A- 381 661
- EP-A- 0 053 603
- EP-A- 0 074 014
- EP-A- 0 233 173
- WO-A-90/11755
- WO-A-91/09834
- DE-A- 2 001 431
- Arzneimitel Forschung/Drug Research, vol., 34 (II), no. 8, 1984, H. G. Hege et al.: "Studies on the metabolism of propafenone", pages843-849, in particular pages 847 and 848, formula 533 (538).
- Arzneimittel Forschung/Drug Research, mvol., 38 (II), no. 9, 1988 R. Neidlein et al.: "Synthesis of glucuronides of propafenone and 5-hydroxypropafenone by sepharose-bound uridine 5 -diphosphoglucuronyl-transferase", pages 1257-1262.

## Beschreibung

Die Erfindung betrifft neue therapeutisch wertvolle Aryloxy-alkylamin-Derivate, deren Herstellung und Arzneimittel, die diese Derivate als Wirkstoff enthalten.

In EP 53 603 werden Derivate des 1-[3-(2-Hydroxy-3-alkylaminopropoxy)-2-thienyl]-3-phenyl-1-propanons beschrieben, welche als solche und in Form ihrer Säureadditionssalze als antiarrhythmische Arzneimittel geeignet sind und teilweise sogar dem Propafenon überlegene Eigenschaften aufweisen.

Ähnliche Verbindungen sind bekannt aus der DE-OS 33 16 155. Auch sie weisen die Hydroxypropoxygruppe auf.

Aus der DE-OS 20 01 431 sind ferner 2-Hydroxy-alkylaminopropoxyphenylpropiophenon-Derivate bekannt, die ebenfalls antiarrhythmische Eigenschaften aufweisen sollen.

Diese bekannten Verbindungen weisen im allgemeinen zufriedenstellende Wirksamkeit bei Verabreichung durch Injektion auf, die Wirksamkeit bei oraler Verabreichung läßt jedoch zu wünschen übrig und erfordert hierfür deutlich höhere Dosierungen zur Erzielung einer befriedigenden Wirksamkeit. Da antiarrhythmische Mittel jedoch in der Regel oral verabreichbar sein sollen, damit eine problemlose Einnahme auch fern von medizinischem Personal durch den Patienten möglich ist, besteht ein Bedarf an Wirkstoffen mit verbesserter Wirksamkeit bei oraler Verabreichung.

Nunmehr wurde überraschend gefunden und hierauf beruht die Erfindung, daß diese Aufgabe gelöst werden kann durch Überführung der freien OH-Gruppe in der 2- Hydroxy-3-aminopropoxygruppe in eine Äther- oder Estergruppe.

Gegenstand der Erfindung sind daher Aryloxy-alkylamin-Derivate der allgemeinen Formel I 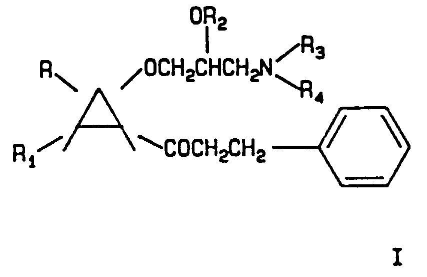
in der A einen Benzol- oder Thiophenring,
R und R₁ unabhängig voneinander jeweils Wasserstoff, Alkyl, Halogen, CF₃ oder Alkoxy,
R₂ Alkyl, Cycloalkyl, Alkenyl, Alkinyl, Alkaryl oder gesättigtes oder ungesättigtes aliphatisches oder aromatisches Acyl, R₃ und R₄ jeweils Wasserstoff, Alkyl, Alkenyl, Alkinyl oder Cycloalkyl mit jeweils bis zu 8 C-Atomen bedeuten, wobei R₃ und R₄ gleich oder verschieden sein können, jedoch nicht gleichzeitig Wasserstoff sind oder R₃ und R₄ zusammen mit dem sie verbindenden Stickstoffatom einen 5 bis 7-gliedrigen gesättigten Ring oder einen gesättigten heterocyclischen Ring bilden, der gegebenenfalls ein Sauerstoff- oder Stickstoffatom als ein weiteres Heteroatom im Ring enthalten kann, wobei ein zusätzliches Stickstoffatom durch einen Alkylrest mit bis zu 3 C-Atomen substituiert sein kann, und ihre Säureadditionssalze.

In den Substituenten R, R₁, R₂, R₃ und R₄ enthalten Alkylgruppen bzw. deren ungesättigte Derivate und der Alkylrest in der Acylgruppe 1 bis 8 C-Atome. Vorzugsweise weisen die Alkyl-, Alkenyl-, Alkinyl-, Alkoxy- und Acylgruppen 1 bis 4 C-Atome auf. Besonders bevorzugt sind diejenigen Verbindungen der allgemeinen Formel I, in denen R und R₁ jeweils H und/oder CH₃ bedeuten.

Unter den Verbindungen der Formel I, in denen A den Thiophenring bedeutet, werden besonders bevorzugt diejenigen mit R = Wasserstoff und R₁ = Methyl. Bei den entsprechenden Verbindungen, in denen A den Benzolring bedeutet, werden diejenigen mit R und R₁ = Wasserstoff besonders bevorzugt.

Unabhängig von der Bedeutung A wird für R₃ die Isobutylgruppe besonders bevorzugt.

Die erfindungsgemäßen Verbindungen enthalten ein asymmetrisches Kohlenstoffatom und liegen daher, wenn keine stereospezifische Synthese angewendet wird, normalerweise in Form des Razemates vor. Die Razemate lassen sich nach üblichen Methoden in die Isomeren trennen. Die optisch aktiven Isomeren sind ebenfalls ein Gegenstand der Erfindung.

Ein weiterer Gegenstand der Erfindung ist ein Verfahren zur Herstellung der Verbindungen der allgemeinen Formel I, welches dadurch gekennzeichnet ist, daß man eine Verbindung der allgemeinen Formel II bzw. IIa 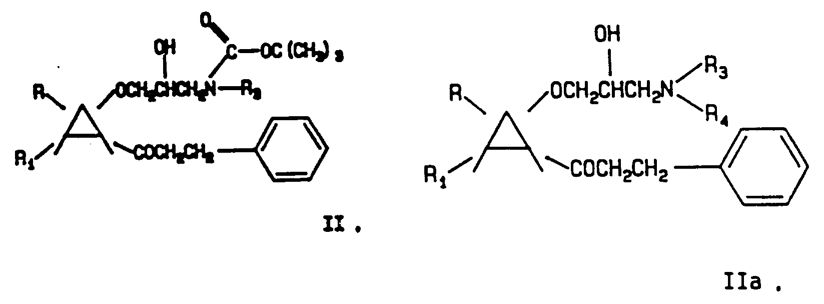
in der A, R, R₁, R₃ und R₄ die zu Formel I angegebene Bedeutung haben, mit einem Alkylüberträger wie einem Alkylhalogenid, Alkylschwefelsäureester oder Alkylsulfonsäureester oder mit einem Acylhalogenid, in Gegenwart von mindestens einem Äquivalent einer Base in einem inerten organischen Lösungsmittel umsetzt, gegebenenfalls die erhaltenen Verbindungen der allgemeinen Formel III 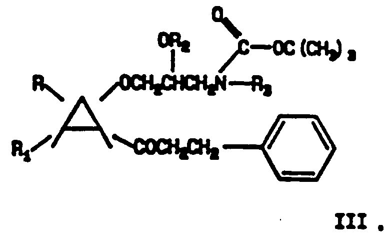
durch Behandlung mit Säure in die entsprechenden Verbindungen der Formel I überführt, in denen R₄ Wasserstoff bedeutet, und die Verbindungen erwünschtenfalls in ein Säureadditionssalz überführt.

Die erfindungsgemäße Umsetzung wird am besten so durchgeführt, daß man eine Verbindung der Formel II oder IIa in einem inerten organischen Lösungsmittel, wie z.B. DMF, THF, Et₂O, Dioxan, löst und anschließend mit mindestens 1 Äquivalent einer starken Base, vorzugsweise einem Alkalihydrid oder -alkoholat versetzt. Die Umsetzungstemperatur liegt zwischen 0°C und 40°C. Anschliessend erfolgt die Umsetzung mit einem Alkylüberträger oder Acylüberträger bei einer Temperatur zwischen 0°C und 70°C. Die Reaktionszeit liegt im allgemeinen zwischen 50 Minuten und 6 Stunden. Durch acidolytische Abspaltung der tert.-Butyloxycarbonyl-Schutzgruppe, insbesondere mit CF₃COOH, werden aus den Verbindungen der allgemeinen Formel III, in einem inerten organischen Lösungsmittel die freien Basen der allgemeinen Formel I erhalten, bei denen R₄ Wasserstoff bedeutet. Die Reaktionsdauer liegt im allgemeinen zwischen 50 Minuten und 2 Stunden, bei einer Reaktionstemperatur zwischen etwa -15°C und -10°C.

Da die Verbindungen der allgemeinen Formel I meist nur schwer kristallisierende Öle sind, die außerdem meist nicht unzersetzt destillierbar sind, empfiehlt es sich, die Reinigung über gut kristallisierende Säureadditionsverbindungen, wie z.B. Hydrochloride vorzunehmen.

Dazu löst man die rohe Base in einem geeigneten Lösungsmittel, z.B. in einem niederen Alkohol oder Ether, gibt eine mindestens äquivalente Menge Säure zu, dampft das Lösungsmittel im Vakuum ab und kristallisiert den Rückstand aus Methanol, Ethanol oder vorzugsweise Aceton, gegebenenfalls unter Zusatz von Ether, um.

Die so erhaltenen Säureadditionssalze können dann in an sich bekannter Weise, z.B. mit Alkalien oder Ionenaustauschern in die freien gereinigten Basen übergeführt werden, von denen sich durch Umsetzung mit organischen oder anorganischen Säuren, insbesondere solchen, die zur Bildung von therapeutisch verwendbaren Salzen geeignet sind, weitere Salze gewinnen lassen.

Infolge der engen Beziehungen zwischen den neuen Verbindungen und deren Salzen gelten die folgenden Ausführungen sinngemäß sowohl für die freien Basen als auch für ihre Salze.

Die Verbindungen der allgemeinen Formel II können, ausgehend von den literaturbekannten Substanzen der Formel IV, in der A, R, R₁ und R₃ die obige Bedeutung haben (EP 0053603; DE-OS 20 01 431) und den käuflichen Reagentien zum Schutz von Aminofunktionen, insbesondere di-tert.-Butyldicarbonat oder tert.-Butylcarbazat (tert.-Butyloxycarbonylazid) gemäß den dem Fachmann geläufigen chemischen Arbeitsmethoden hergestellt werden, z.B. wie folgt:

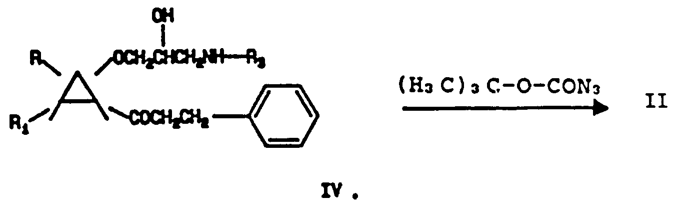

Die Säureadditionssalze der Endverbindungen können in an sich bekannter Weise, beispielsweise durch Zusetzen eines Alkalis oder durch Ionenaustauscher in die freien Basen übergeführt werden. Andere Salze können daraus durch Umsetzen mit anorganischen oder organischen Säuren, insbesondere solchen, die zur Bildung von therapeutisch verwendbaren und pharmazeutisch annehmbaren Salzen geeignet sind, gebildet werden.

Beispiele für pharmazeutisch verträgliche Salze sind die Salze von Halogenwasserstoffsäuren, Schwefelsäuren, Phosphorsäuren, Salpetersäure, Perchlorsäure sowie von aliphatischen, alicyclischen, aromatischen oder heterocyclischen Carbonsäuren- oder Sulfonsäuren wie

| | |
|---|---|
| Ameisensäure | |
| Glutarsäure | |
| Essigsäure | Hydroxymaleinsäure |
| Propionsäure | Phenylessigsäure |
| Buttersäure | Benzoesäure |
| Isovaleriansäure | p-Amino-benzoesäure |
| Bernsteinsäure | p-Hydroxybenzoesäure |
| Glykolsäure | Anthranilsäure |
| Milchsäure | Salicylsäure |
| Brenztraubensäure | Methansulfonsäure |
| Glycerinsäure | Äthansulfonsäure |
| Äpfelsäure | Hydroxyäthansulfonsäure |
| Weinsäure | Äthylensulfonsäuren |
| Zitronensäure | Halogenbenzolsulfonsäuren |
| Ascorbinsäure | Toluolsulfonsäure |
| Malonsäure | Naphtalinsulfonsäuren |
| Maleinsäure | Sulfanilsäure |
| Fumarsäure | Methionin |
| Oxalsäure | Nikotinsäure |
| Tryptophan | |
| Lysin | |
| Arginin | |
| N-Acetylcystein | |
| Schleimsäure | |
| Ev. Pikrinsäure zur Reinigung | |

Aus der EP 0 362 707 sind Hydroxypropafenonglyceride bekannt, die ebenfalls als Wirkstoffe für Antiarrhythmika verwendbar sein sollen. Für diese Glyceridderivate wird ebenfalls eine orale Verabreichbarkeit angegeben. In dieser älteren, aber nicht vorveröffentlichten Anmeldung werden jedoch nur Substanzen beschrieben, die von den erfindungsgemäßen Verbindungen verschieden sind.

In der eigenen älteren, nicht vorveröffentlichten EP 0 381 661 werden ebenfalls Verbindungen beschrieben, die sich von den Verbindungen der vorliegenden Erfindung schon allein dadurch unterscheiden, daß sie an der für R₂ angegebenen Position Wasserstoff enthalten, was gemäß vorliegender Erfindung ausgeschlossen ist.

Glukuronide von Propafenon und 5-Hydroxypropafenon sind schon beschrieben in Arzneimittelforschung/Drug Research 38 (II), Nr. 9 (1988) 1257 bis 1262. Für diese Verbindungen wird angegeben, daß sie zur Untersuchung des Metabolismus von Propafenon selbst interessant sind. Über die pharmakologischen Eigenschaften dieser Verbindungen, die von denen der Erfindung deutlich verschieden sind, läßt sich daraus nichts entnehmen.

Es können jedoch auch andere Säuren verwendet werden.

Die neuen Verbindungen der Formel I und ihre pharmazeutisch verwendbaren Salze zeigen hervorragende antiarrhythmische Eigenschaften, insbesondere bei oraler Verabreichung.

Ein weiterer Gegenstand der Erfindung ist daher ein Arzneimittel, welches gekennzeichnet ist durch einen Gehalt an wenigstens einer Verbindung der allgemeinen Formel I oder einem pharmakologisch verträglichen Salz davon in Kombination mit üblichen galenischen Hilfs- und/oder Trägerstoffen. Ein derartiges Arzneimittel ist zur Behandlung von Krankheiten des Herz-Kreislauf-Systems geeignet, insbesondere als Antiarrhythmikum.

Aufgrund dieser pharmakologischen Eigenschaften können die neuen Verbindungen alleine oder in Mischung mit anderen Wirksubstanzen in Form üblicher galenischer Zubereitung bei Erkrankungen, die durch Herzrhythmusstörungen, wie z.B. Tachykardie, verursacht werden, als Medikament verwendet werden.

Unter den Arten von Tachykardien, die mit den erfindungsgemäßen Verbindungen behandelt werden können, seien supraventrikuläre und ventrikuläre Tachykardien, supraventrikuläre und ventrikuläre Ektopien und "Reentry"-Tachykardien genannt.

Die erfindungsgemäßen Arzneimittel enthalten die erfindungsgemäßen Verbindungen der allgemeinen Formel I in Mischung mit einem für die enterale oder parenterale Applikation geeigneten pharmazeutischen, organischen oder anorganischen Trägermaterial, beispielsweise Wasser, Gelatine, Gummi arabicum, Milchzucker, Stärke, Magnesiumstearat, Talk, pflanzliche Öle, Polyalkylenglykole, Vaseline oder dergleichen.

Die Arzneimittel können in fester Form z.B. als Tabletten, Filmtabletten, Dragees, Suppositorien, Kapseln, Mikrokapseln, Pflaster oder in flüssiger Form z.B. als Lösungen, Injektionslösungen, Suspensionen oder Emulsionen oder in Zusammensetzungen mit verzögerter Freigabe des Wirkstoffes vorliegen.

Gegebenenfalls sind sie sterilisiert und/oder enthalten Hilfsstoffe, wie Konservierungs-, Stabilisierungs- oder Emulgiermittel, Salze zur Veränderung des osmotischen Druckes oder Puffer.

Insbesondere können derartige pharmazeutische Präparate die erfindungsmäßigen Verbindungen in Kombination mit anderen therapeutisch wertvollen Stoffen enthalten. Mit diesen können die erfindungsgemäßen Verbindungen zusammen mit den oben angegebenen Hilfs- und/oder Trägerstoffen zu Kombinationspräparaten formuliert werden.

Die neuen Verbindungen sind zweckmäßigerweise in den erfindungsgemäßen Arzneimitteln in einem Anteil von etwa 10 bis 800 mg/Tablette vorhanden.

Eine geeignete Dosis zur oralen Verabreichung der neuen Verbindung beträgt etwa 1 bis 20 mg/kg pro Tag, jedoch kommen je nach dem Zustand des zu behandelnden Patienten auch andere Dosen in Frage. Die neuen Verbindungen können in mehreren Dosen und auf oralem Weg verabreicht werden.

### Pharmakologische Eigenschaften der erfindungsgemäßen Verbindung

Als repräsentative Verbindung wurde 1-[3-(2-Methoxy-3-(2-methylpropylamino)-propoxy)-4-methyl-2-thienyl]-3-phenyl-1-propanon, Hydrochlorid (Derivat 1) hinsichtlich seiner antiarrhythmischen Wirksamkeit untersucht. Die Verlängerung der effektiven Refraktärperiode wurde als Kriterium zur Beurteilung der antiarrhythmischen Wirksamkeit herangezogen. Als Vergleichssubstanzen dienten 1-[3-(2-Hydroxy-3-isobutylaminopropoxy)-4-methyl-2-thienyl]-3-phenyl-1-propanon, Hydrochlorid (interne Bezeichnung: LG 83-6-05) und Propafenon.

Die Refraktärzeiten verschiedener Abschnitte des Reizleitungssystems und vom atrialen und ventrikukärem Myokard wurden mittels modifizierter Langendorff-Methode und einem hochauflösenden Oberflächen EKG gemessen.

Für die Versuchsanordnung wurden Herzen von 300 - 400 g schweren Meerschweinchen gewählt, die mit Sauerstoff (95 %) und CO₂ (5 %) angereicherter Tyrode perfundiert wurden (Perfusionsrate 4 bis 6 ml/min.)

Zwei Silberelektroden wurden an der Herzoberfläche des spontan schlagenden Herzens epikardial angelegt und zwar an der Klappenebene. Die Equilibrierdauer betrug 30 Minuten. Die Ergebnisse zeigt Tabelle 1.

**Tabelle 1**

| Veränderung der Überleitungszeit in %, hervorgerufen durch 1 µM Substanz in Abhängigkeit von der Perfusionsdauer. | | | | |
|---|---|---|---|---|
| | 15 min | 30 min | 45 min | 60 min |
| | | | | |

| Propafenon (Vergleich) | | | | |
|---|---|---|---|---|
| AH-Zeit | **109±2 | **111±2 | **113±2 | **117±4 |
| HV-Zeit | **113±3 | **122±7 | *124±11 | *125±10 |
| QRS-Dauer | **115±3 | **120±5 | **122±6 | *123±7 |

| LG 83-6-05 (Vergleich) | | | | |
|---|---|---|---|---|
| AH-Zeit | **113±1 | **116±1 | **117±2 | **120±2 |
| HV-Zeit | **125±6 | **147±6 | *139±16 | **153±10 |
| QRS-Dauer | **114±14 | 116±8 | *118±8 | *116±6 |

| Derivat 1 | | | | |
|---|---|---|---|---|
| AH-Zeit | **119±2 | **127±7 | *132±10 | *147±16 |
| HV-Zeit | **111±2 | *118±5 | 133±17 | 171±49 |
| QRS-Dauer | **138±2 | **179±14 | **176±16 | **185±22 |

Derivat 1 zeigte im Vergleich zu LG 83-6-05 und Propafenon eine wesentlich (signifikant) größere Verlängerung der Überleitungszeiten, sowohl der AH-Zeit als auch der HV-Zeit sowie eine Verlängerung des QRS-Komplexes.

Als weitere erfindungsgemäße Substanz wurde 2-(2-Methoxy-3-propylamino-propoxy)-3-phenyl-propiophenon, Hydrochlorid (Derivat 2) getestet und zwar am Modell der Ouabain induzierten Arrhythmie am Meerschweinchen nach oraler Verabreichung. Dabei dienten wiederum LG 83-6-05 und Propafenon als Vergleichssubstanzen.

Den narkotisierten Tieren wird eine konstante Menge Ouabain (20 µg/kg/min) intravenös infundiert und der Zeitpunkt registriert, bei dem die erste ventrikuläre Extrasystole im EKG auftritt.

Durch Vorbehandlung der Versuchstiere mit steigenden Dosen verlängert sich die Zeit bis zum Auftreten der ersten ventrikulären Extrasystolen bzw. erhöht sich die Dosis Ouabain. Aus Dosis und Zeit läßt sich eine Dosiswirkungskurve erstellen.

Nachstehende Tabelle 2 zeigt die Wirkung der oralen Vorbehandlung gegenüber der Ouabain-induzierten Arrhythmie des Meerschweinchens. Zeitpunkt des Auftretens der ersten Extrasystole ausgedrückt in % der Kontrolle (Mittelwerte).

**Tabelle 2**

| | % nach 40 mg/kg | % nach 80 mg/kg |
|---|---|---|
| Propafenon (Vergleich) | 117 | 145 |
| LG 83-6-05 (Vergleich) | 101 | 124 |
| Derivat 2 | 121 | 174 |

Die vorstehend beschriebenen Ergebnisse zeigen, daß die erfindungsgemäßen Derivate sowohl bezüglich ihrer elektrophysiologischen Eigenschaften am isolierten Herzen wie auch am Ganztier gegenüber den vergleichbaren Standardsubstanzen Propafenon und LG 83-6-05 eine wesentlich stärkere Wirkung aufweisen.

Die folgenden Beispiele erläutern die Erfindung näher.

### Beispiel 1

### 1-[3-(2-Methoxy-3-(tert.-butyloxycarbonyl-2-methylpropylamino)-propoxy)-4-methyl-2-thienyl]-3-phenyl-1-propanon

15 g (31,5 mMol) 1-[3-(2-Hydroxy-3-(tert.-butyloxycarbonyl-2-methylpropylamino)-propoxy)-4-methyl-2-thienyl]-3-phenyl-1-propanon werden in 140 ml abs. Ether gelöst und bei 0°C mit 0,80 g (33,3 mMol) Natriumhydrid versetzt. Danach wird 55 Minuten unter Rückfluß erhitzt. Anschließend wird das Reaktionsgemisch auf 0°C abgekühlt und bei dieser Temperatur eine Lösung von 4,4 g (34,9 mMol) Dimethylsulfat in 10 ml abs. Ether zugetropft. Nach 65 Minuten bei 0°C bis 5°C wird das Reaktionsgemisch auf 2N HCl geleert. Die Phasen werden getrennt und die H₂O-Phase dreimal mit CH₂Cl₂ ausgeschüttelt. Die vereinigten organischen Phasen werden über Na₂SO₄ getrocknet und eingedampft. Es werden 22 g eines orangegelben Öles erhalten, welches säulenchromatographisch gereinigt wird.
Säulenchromatographie: Kieselgel, PE/EtOH = 8:1 bzw. CH₂Cl₂/EtOH = 60 : 1
Ausbeute: 13,0 g farbloses Öl (84 % d. Th.)
¹H-NMR (CDCl₃):
δ (ppm): 7,24 - 7,20 (m; 5H; Bz-H); 7,10 (d; 1H; Th-H₅); 4,24 - 3,93 (m; 3H; -OCH₂CH); 3,50 - 3,14 (h; 4H; -CH₂NCH₂); 3,34 (s; 3H; -OCH₃); 2,98 - 2,69 (m; 4H; -CH₂CH₂); 2,17 (d; 3H; Th-CH₃); 1,83 - 1,59 (m; 1H; -CH); 1,44 (s; 9H; -OC(CH₃)₃); 0,86 (d; 6H; -(CH₃)₂)

### 1-[3-(2-Methoxy-3-(2-methylpropylamino)-propoxy)-4-methyl-2-thienyl]-3-phenyl-1-propanon, Hydrochlorid

Eine Lösung von 10 g (20,4 mMol) 1-[3-(2-Methoxy-3-(tert.-butyloxycarbonyl-2-methylpropylamino)-propoxy)-4-methyl-2-thienyl]-3-phenyl-1-propanon in 50 ml absolutem Methylenchlorid wird auf -15°C abgekühlt und mit 96,9 g (0,850 Mol) Trifluoressigsäure (FLUKA, Art.Nr. 91700) versetzt. Anschließend wird bei -15°C bis -10°C gerührt. Nach 2 Stunden wird unter Eiskühlung mit einer gesättigten Natriumcarbonatlösung neutralisiert und die Phasen getrennt. Die H₂O-Phase wird noch zweimal mit Methylenchlorid ausgeschüttelt und die vereinigten organischen Phasen über Na₂SO₄/AK getrocknet und eingedampft. Es werden 6,8 g eines gelben Öles (85 % d. Th.) erhalten.

Das Rohprodukt wird in ca. 100 ml abs. Ether gelöst und unter Kühlung mit überschüssiger etherischer Salzsäure versetzt. Das zunächst leicht schmierig anfallende Hydrochlorid wird kristallisiert und die hellgelben Kristalle abgesaugt. Die so erhaltenen ca. 7,2 g Rohprodukt werden aus wenig Aceton unter Zusatz von Ether umkristallisiert.
Ausbeute: 5,3 g farblose Kristalle (61 % d.Th.)
Fp.: 102 - 104°C

| Mikroelementaranalyse: C₂₂H₃₂ClNO₃S (426,02) | | | |
|---|---|---|---|
| | C | H | N |
| ber.: | 62,03 | 7,57 | 3,29 |
| gef.: | 62,02 | 7,54 | 3,25 |

¹H-NMR (CDCl₃):
δ (ppm): 7,27 - 7,21 (m; 5H; Bz-H); 7,14 (d; 1H; Th-H₅); 4,30 - 3,95 (m; 3H; -OCH₂CH); 3,50 (s; 3H; -OCH₃); 3,37 - 3,12 (h; 4H; -CH₂NCH₂); 2,92 - 2,68 (m; 4H; -CH₂CH₂); 2,17 (d; 3H; Th-CH₃); 1,98 - 1,80 (m; 1H; -CH); 1,05 (d; 6H; -(CH₃)₂)

### Beispiel 2

### 2-[2-Methoxy-3-(tert.-butyloxycarbonyl-propylamino)-propoxy]-3-phenyl-propiophenon

5 g (11,3 mMol) 2-[2-Hydroxy-3-(tert.-butyloxycarbonyl-propylamino)-propoxy]-3-phenyl-propiophenon werden in 50 ml abs. DMF gelöst und bei 0°C mit 0,30 g (12,5 mMol) Natriumhydrid versetzt. Danach wird 70 Minuten bei Raumtemperatur gerührt. Anschließend wird das Reaktionsgemisch auf 0°C abgekühlt und eine Lösung von 1,60 g (12,5 mMol) Dimethylsulfat in 5 ml abs. DMF zugetropft. Nach 40 Minuten bei 0° bis 5°C wird das Reaktionsgemisch auf 2N HCl geleert. Die Phasen werden getrennt und die H₂O-Phase dreimal mit CH₂Cl₂ extrahiert. Die vereinigten organischen Phasen werden nach dem Trocknen über Na₂SO₄ eingedampft. Es werden 4,5 g eines orangen Öles erhalten, welches säulenchromatographisch gereinigt wird. Säulenchromatographie: Kieselgel, Bz : EE = 14 : 1 Das gereinigte Öl kristallisiert im Tiefkühlschrank.
Ausbeute: 2,2 g farblose Kristalle (43 % d. Th.)
Fp.: 53 - 55°C
¹H-NMR (CDCl₃):
δ (ppm): 7,62 - 6,96 (m; 9H; Bz-H): 4,07 - 3,68 (m; 3H; -OCH₂CH); 3,38 - 3,09 (h; 8H; -CH₂NCH₂ und -CH₂CH₂); 3,30 (s; 3H; -OCH₃); 1,70 - 1,40 (m; 2H; -CH₂); 1,43 (s; 9H; -OC(CH₃)₃); 0,90 (t; 3H; -CH₃)

### 2-(2-Methoxy-3-propylamino-propoxy)-3-phenyl-propiophenon

8,2 g (18,0 mMol) 2-[2-Methoxy-3-(tert.-butyloxycarbonyl-propylamino)-propoxy]-3-phenyl-propiophenon werden in 150 ml abs. CH₂Cl₂ gelöst und auf -15°C abgekühlt. Danach wird mit 122,9 g (1,08 Mol) Trifluoressigsäure versetzt und 90 Minuten bei einer Temperatur zwischen -15°C und -10°C gerührt. Anschließend wird unter Eiskühlung mit einer gesättigten Natriumcarbonatlösung neutralisiert und die Phasen getrennt. Die H₂O-Phase wird noch zweimal mit CH₂Cl₂ ausgeschüttelt und die vereinigten organischen Phasen über Na₂SO₄/AK getrocknet und eingedampft. Es werden 5,5 g gelbes Öl (86 % d. Th.) erhalten. Das Rohprodukt wird zur Hydrochloridbildung in abs. Ether gelöst und die Lösung unter Kühlung mit etherischer Salzsäure versetzt. Das kristalline Hydrochlorid wird abgesaugt aus Aceton/Ether umkristallisiert und im Vakuum getrocknet.
Ausbeute: 5,0 g farblose Kristalle (71 % d. Th.)
Fp.: 109 - 111°C

| Mikroelementaranalyse: C₂₂H₃₀ClNO₃ (391,94) | | | |
|---|---|---|---|
| | C | H | N |
| ber.: | 67,42 | 7,72 | 3,57 |
| gef.: | 67,48 | 7,76 | 3,65 |

¹H-NMR (CDCl₃):
δ (ppm): 7,66 - 6,91 (m; 9H; Bz-H); 4,20 - 3,61 (m; 3H; -OCH₂CH); 3,49 (s; 3H; -OCH₃); 3,29 - 3,04 (m; 8H; -CH₂NCH₂ und -CH₂CH₂); 1,85 - 1,53 (m; 2H; -CH₂); 0,93 (t; 3H; -CH₃)

### Beispiel 3

### 1-[3-(2-Methoxy-3-diisopropylaminopropoxy)-4-methyl-2-thienyl]-3-phenyl-1-propanon

4,44 g (11,0 mMol) 1-[3-(2-Hydroxy-3-diisopropylaminopropoxy)-4-methyl-2-thienyl]-3-phenyl-1-propanon werden in 45 ml abs. Dioxan gelöst und bei Raumtemperatur mit 0,27 g (11,3 mMol) NaH versetzt. Danach wird 45 Minuten auf 40°C erwärmt. Anschließend wird das Reaktionsgemisch auf 20°C abgekühlt und eine Lösung von 1,52 g (12,1 mMol) Dimethylsulfat in 2 ml abs. Dioxan zugetropft. Nach 5 h bei Raumtemperatur wird eingedampft und der Rückstand zwischen einer gesätt. Na₂CO₃-Lösung und Ether verteilt. Die Phasen werden getrennt und die H₂O-Phase dreimal mit Ethylacetat extrahiert. Die vereinten organischen Phasen werden über Na₂SO₄ getrocknet und eingedampft. Es werden 4,7 g gelbes Öl erhalten, welches säulenchromatographisch gereinigt wird.
Säulenchromatographie: Kieselgel imprägniert, PE/EtOAc = 4 : 1
Die erhaltenen 2,3 g ölige freie Base werden in abs. Ether mit etherischer Salzsäure zum glasig amorphen Hydrochlorid umgesetzt.
Ausbeute: 2,2 g farbloses Pulver (44 % d.Th.)
Fp.: 38 - 42°C

| Mikroelementaranalyse: C₂₄H₃₆ClNO₃S.0,42H₂O (stark hygroskopisch) (461,64) | | | |
|---|---|---|---|
| | C | H | N |
| ber.: | 62,44 | 7,86 | 3,03 |
| gef.: | 62,20 | 8,07 | 3,11 |

¹H-NMR (CDCl₃)
δ(ppm): 7,26-7,20 (m; 5H; Bz-H); 7,02 (d; 1H, ThH₅); 4,40-4,08 (m; 3H;-OCH₂CH); 3,78-3,61 (m; 3H;-CH₂NCH); 3,52 (s; 3H;-OCH₃); 3,42-3,05 (h; 5H;-CH₂CH₂;-NCH); 2,24 (d; 3H; ThCH₃); 1,57-1,28 (m;12H; -(CH₃)₂)

### Beispiel 4

### 2-(2-Methoxy-3-diisopropylamino-propoxy)-3-phenyl-propiophenon

4,78 g (12,5 mMol) 2-(2-Hydroxy-3-diisopropylamino-propoxy)-3-phenyl-propiophenon werden in 48 ml abs. Dioxan gelöst und mit 0,30 g (12,5 mMol) Natriumhydrid versetzt. Danach wird 2 Stunden bei 40°C gerührt. Anschließend wird auf Raumtemperatur abgekühlt und bei dieser Temperatur 1,75 g (13,9 mMol) Dimethylsulfat gelöst in 4 ml abs. Dioxan zugetropft. Nach 1,5 h bei 40°C wird eingedampft. Der Rückstand wird zwischen einer Na₂CO₃-Lösung und Ether verteilt; die H₂O-Phase wird mit Ether ausgeschüttelt. Nach dem Trocknen über Na₂SO₄ und Eindampfen werden 5,3 g orangegelbes Öl erhalten, welches säulenchromatographisch gereinigt wird.
Säulenchromatographie:
Kieselgel imprägniert, PE/EtOAc = 4 : 1 bzw.
Kieselgel CH₂Cl₂/EtOH = 15 : 1
Ausbeute: 2,7 g blaßgelbes Öl (54,5 % d. Th.)

| Mikroelementaranalyse: C₂₅H₃₅NO₃ (397,56) | | | |
|---|---|---|---|
| | C | H | N |
| ber.: | 75,53 | 8,87 | 3,52 |
| gef.: | 75,72 | 8,94 | 3,49 |

¹H-NMR (CDCl₃)
δ(ppm): 7,76-6,99 (m; 9H; Bz-H); 4,35-4,07 (m; 2H; -OCH₂); 3,59-3,37 (m; 3H;-NCH₂;-CH); 3,29 (s; 3H; -OCH₃); 3,11-2,56 (h;6H;-CH₂CH₂;-N(CH)₂); 0,97 (d; 12H;-(CH₃)₂)
Die Ausgangsprodukte können wie folgt hergestellt werden:

### 1-[3-(2-Hydroxy-3-(tert.-butyloxycarbonyl-2-methylpropylamino)-propoxy-4-methyl-2-thienyl]-3-phenyl-1-propanon

Zu einer auf 10°C gekühlten Lösung von 11,2 g (29,8 mMol) 1-/3-(2-Hydroxy-3-(2-methylpropylamino)-propoxy)-4-methyl-2-thienyl/-3-phenyl-1-propanon in 230 ml Dioxan werden 7,1 g (32,5 mMol) di-tert.-Butyl-dicarbonat (FLUKA; 34659) zugegeben. Nach dem Zutropfen von 15 ml 0,5 N NaOH wird das Reaktionsgemisch auf 2°C abgekühlt und 40 Minuten bei dieser Temperatur gerührt. Danach wird unter Vakuum eingedampft, der Rückstand in CH₂Cl₂ und H₂O aufgenommen, die Phasen getrennt und die H₂O-Phase noch dreimal mit CH₂Cl₂ ausgeschüttelt Nach der Trocknung über Na₂SO₄ und Eindampfen werden 13,5 g hellgelbes Öl erhalten, welches im Tiefkühlschrank kristallisiert. Die Kristalle werden ohne weitere Reinigung direkt in die nächste Stufe eingesetzt.
Ausbeute: 13,2 g farblose Kristalle (93 % d. Th.)
Fp.: 47 - 50°C
¹H-NMR (CDCl₃):
(ppm): 7,24 - 7,18 (m;5H; Bz-H); 7,08 (d; 1H; Th-H₅); 4,19 - 3,90 (m; 3H; -OCH₂CH); 3,32 - 3,09 (h; 4H; - CH₂NCH₂); 2,90 - 2,75 (m; 4H; -CH₂CH₂); 2,54 - 2,43 (breit; 1H; OH); 2,20 (d; 3H; Th-CH₃); 1,79 - 1,65 (m; 1H; -CH); 1,48 (s; 9H; -OC(CH₃)₃); 0,89 (d; 6H; -(CH₃)₂)

### 2-[2-Hydroxy-3-(tert.-butyloxycarbonyl-propylamino)-propoxy]-3-phenyl-propiophenon

Zu einer auf 10°C gekühlten Lösung von 20 g (58,6 mMol) 2-(2-Hydroxy-3-propylamino-propoxy)-3-phenyl-propiophenon in 230 ml Dioxan werden 15,3 g (70,1 mMol) di-tert.-Butyldicarbonat zugegeben. Nach dem Zutropfen von 117 ml 0,5 N NaOH wird auf 2°C abgekühlt und 50 Minuten gerührt. Danach wird unter Vakuum eingedampft, der Rückstand in H₂O und CH₂Cl₂ aufgenommen, die Phasen getrennt und die H₂O-Phase noch dreimal mit CH₂Cl₂ ausgeschüttelt. Nach Trocknung über Na₂SO₄ und Eindampfen werden 24,5 g blaßgelbes Öl erhalten, welches im Tiefkühlschrank kristallisiert und ohne weitere Reinigung in die nächste Stufe eingesetzt wird.
Ausbeute: 24,5 g hellgelbe Kristalle (95 % d. Th.)
Fp.: 86 - 89°C
¹H-NMR (CDCl₃):
δ (ppm): 7,54 - 6,99 (m; 9H; Bz-H); 4,03 - 3,72 (m; 3H; -OCH₂CH); 3,37 - 3,01 (h; 8H; -CH₂CH₂ und -CH₂NCH₂); 1,70 - 1,42 (m; 2H; -CH₂); 1,45 (s; 9H; -OC(CH₃)₃); 0,85 (t; 3H; -CH₃)

### 1-[3-(2-Hydroxy-3-diisopropylamino-propoxy)-4-methyl-2-thienyl]-3-phenyl-1-propanon

25,0 g (82,7 mMol) 1-[3-(2,3-Epoxypropoxy)-4-methyl-2-thienyl]-3-phenyl-1-propanon werden mit 84 ml Diisopropylamin versetzt und 10 h unter Rückfluß erhitzt. Danach wird eingedampft und der Rückstand zwischen einer ges. Na₂CO₃-Lösung und Ether verteilt. Die Phasen werden getrennt und die wäßrige Phase mit Ether extrahiert. Anschließend wird die organische Phase mit 2N HCl geschüttelt; die HCl-Phase unter Eiskühlung mit 4N HCl neutralisiert und mit Ether extrahiert. Nach der Trocknung über Na₂SO₄ werden 18,6 g Öl erhalten, welches chromatographisch gereinigt wird.
Säulenchromatographie: Kieselgel, CH₂Cl₂/EtOH = 12 : 1
Ausbeute: 14,8 g gelbes Öl (4,44 % d.Th.)
Fp.: 66 - 69°C (Hydrochlorid)
¹H-NMR (CDCl₃)
δ (ppm): 7,23-7,19 (m; 5H; Bz-H); 7,10 (d; 1H; Th-H₅); 4,32-4,00 (m; 3H; -OCH₂CH); 3,47-2,79 (h; 8H; -CH₂CH₂; -CH₂N(CH)₂); 2,25 (d; 3H; Th-CH₃); 1,46-1,10 (m; 12H;-(CH₃)₂)

### 2-(2-Hydroxy-3-diisopropylamino-propoxy)-3-phenyl-propiophenon

10,0 g (35,4 mMol) 2-[2-(2,3-Epoxypropoxy)]-3-phenyl-propiophenon werden in 36 ml Diisopropylamin gelöst und anschließend 10,5 h unter Rückfluß erhitzt. Danach wird eingedampft und der Rückstand zwischen einer gesättigten Na₂CO₃-Lösung und Ether verteilt. Die etherische Phase wird erschöpfend mit 2N HCl extrahiert, die HCl-Phase 1 x mit Ether gewaschen und 4 x mit CH₂Cl₂ ausgeschüttelt. Die CH₂Cl₂-Phase wird über Na₂SO₄ getrocknet und eingedampft. Es werden 7,6 g elfenbeinfarbene Kristalle erhalten. Das Rohprodukt wird aus Diisopropylether/Aceton umkristallisiert.
Ausbeute: 6,2 g farblose Kristalle (45,6 % d. Th.)
Fp.: 125 - 129°C
¹H-NMR (CDCl₃)
δ (ppm): 7,70-6,97 (m; 9H; Bz-H); 4,13-3,91 (m; 3H;-OCH₂CH); 3,48-3,24 (m; 2H;-NCH₂); 3,14-2,42 (m; 6H; -CH₂CH₂; -N(CH)₂); 1,06-0,94 (m; 12H;-(CH₃)₂)

## Patentansprüche (Patentansprüche für folgende(n) Vertragsstaat(en): AT, BE, CH, DE, DK, FR, GB, IT, LI, LU, NL, SE)

1. Aryloxy-alkylamin-Derivate der allgemeinen Formel I 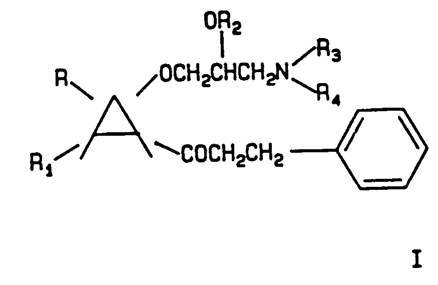 in der A einen Benzol- oder Thiophenring,
R und R₁ unabhängig voneinander jeweils Wasserstoff, Alkyl, Halogen, CF₃ oder Alkoxy,
R₂ Alkyl, Cycloalkyl, Alkenyl, Alkinyl, Alkaryl oder gesättigtes oder ungesättigtes aliphatisches oder aromatisches Acyl, R₃ und R₄ jeweils Wasserstoff, Alkyl, Alkenyl, Alkinyl oder Cycloalkyl mit jeweils bis zu 8 C-Atomen bedeuten, wobei R₃ und R₄ gleich oder verschieden sein können, jedoch nicht gleichzeitig Wasserstoff sind oder R₃ und R₄ zusammen mit dem sie verbindenden Stickstoffatom einen 5 bis 7-gliedrigen gesättigten Ring oder einen gesättigten heterocyclischen Ring bilden, der gegebenenfalls ein Sauerstoff- oder Stickstoffatom als ein weiteres Heteroatom im Ring enthalten kann, wobei ein zusätzliches Stickstoffatom durch einen Alkylrest mit bis zu 3 C-Atomen substituiert sein kann, und ihre Säureadditionssalze.

2. Derivat nach Anspruch 1,
**dadurch gekennzeichnet,**
daß Alkyl, Alkenyl, Alkinyl, Alkoxy und Acyl 1 bis 4 C-Atome aufweisen.

3. Derivat nach Anspruch 1 oder 2,
**dadurch gekennzeichnet,**
daß R, R₁ = H und/oder CH₃ bedeuten.

4. Derivat nach Anspruch 3,
**dadurch gekennzeichnet,**
daß A den Thiophenring, R Wasserstoff und R₁ Methyl bedeuten.

5. Derivat nach Anspruch 3,
**dadurch gekennzeichnet,**
daß A den Benzolring und R und R₁ Wasserstoff bedeuten.

6. Derivat nach den Ansprüchen 2 bis 5,
**dadurch gekennzeichnet,**
daß R₃ Isobutyl bedeutet.

7. 1-[3-(2-(Methoxy-3-(2-methylpropylamino)-propoxy)-4-methyl-2-thienyl]-3-phenyl-1-propanon.

8. 1-[3-(2-Methoxy-3-(2-methylpropylamino)-propoxy)-4-methyl-2-thienyl]-3-phenyl-1-propanon, Hydrochlorid.

9. 2-(2-Methoxy-3-propylamino-propoxy)-3-phenyl-propiophenon.

10. 2-(2-Methoxy-3-propylamino-propoxy)-3-phenyl-propiophenon, Hydrochlorid.

11. Verfahren zur Herstellung eines Derivats nach einem der Ansprüche 1 bis 10, **dadurch gekennzeichnet,** daß man eine Verbindung der allgemeinen Formel II bzw. IIa 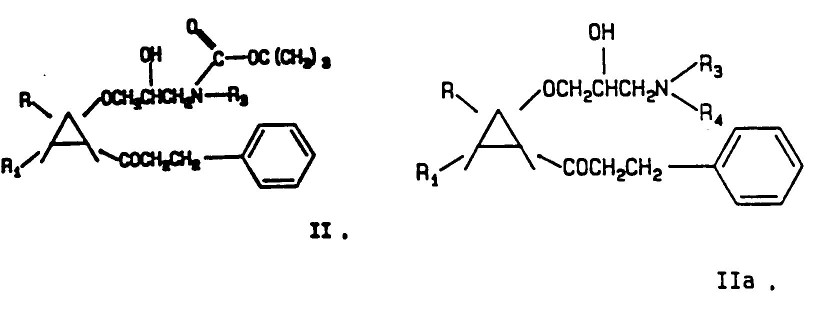 in der A, R, R₁, R₃ und R₄ die zu Formel I angegebene Bedeutung haben, mit einem Alkylüberträger wie einem Alkylhalogenid, Alkylschwefelsäureester oder Alkylsulfonsäureester oder mit einem Acylhalogenid, in Gegenwart von mindestens einem Äquivalent einer Base in einem inerten organischen Lösungsmittel umsetzt, gegebenenfalls die erhaltenen Verbindungen der allgemeinen Formel III 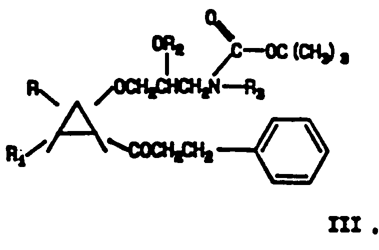 durch Behandlung mit Säure in die entsprechenden Verbindungen der Formel I überführt, in denen R₄ Wasserstoff bedeutet, und die Verbindungen erwünschtenfalls in ein Säureadditionssalz überführt.

12. Arzneimittel,
**gekennzeichnet durch**
einen Gehalt an wenigstens einer Verbindung der allgemeinen Formel I oder einem pharmakologisch verträglichen Salz davon in Kombination mit üblichen galenischen Hilfs- und/oder Trägerstoffen.

13. Arzneimittel nach Anspruch 12 zur Behandlung von Krankheiten des Herz-Kreislaufsystems, insbesondere Antiarrhythmikum.

## Patentansprüche (Patentansprüche für folgende(n) Vertragsstaat(en): ES)

1. Verfahren zur Herstellung von Aryloxy-alkylamin-Derivaten der allgemeinen Formel I 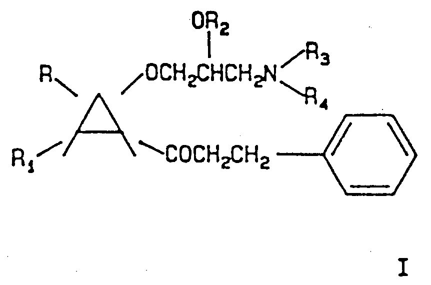 in der A einen Benzol- oder Thiophenring, R und R₁ unabhängig voneinander jeweils Wasserstoff, Alkyl, Halogen, CF₃ oder Alkoxy, R₂ Alkyl, Cycloalkyl, Alkenyl, Alkinyl, Alkaryl oder gesättigtes oder ungesättigtes aliphatisches oder aromatisches Acyl, R₃ und R₄ jeweils Wasserstoff, Alkyl, Alkenyl, Alkinyl oder Cycloalkyl mit jeweils bis zu 8 C-Atomen bedeuten, wobei R₃ und R₄ gleich oder verschieden sein können, jedoch nicht gleichzeitig Wasserstoff sind oder R₃ und R₄ zusammen mit dem sie verbindenden Stickstoffatom einen 5 bis 7-gliedrigen gesättigten Ring oder einen gesättigten heterocyclischen Ring bilden, der gegebenenfalls ein Sauerstoff- oder Stickstoffatom als ein weiteres Heteroatom im Ring enthalten kann, wobei ein zusätzliches Stickstoffatom durch einen Alkylrest mit bis zu 3 C-Atomen substituiert sein kann, und ihrer Säureadditionssalze,
**dadurch gekennzeichnet,**
daß man eine Verbindung der allgemeinen Formel II bzw. IIa 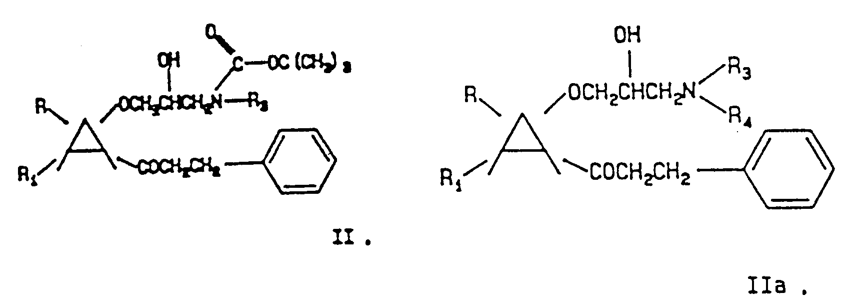 in der A, R, R₁, R₃ und R₄ die zu Formel I angegebene Bedeutung haben, mit einem Alkylüberträger wie einem Alkylhalogenid, Alkylschwefelsäureester oder Alkylsulfonsäureester oder mit einem Acylhalogenid, in Gegenwart von mindestens einem Äquivalent einer Base in einem inerten organischen Lösungsmittel umsetzt, gegebenenfalls die erhaltenen Verbindungen der allgemeinen Formel III 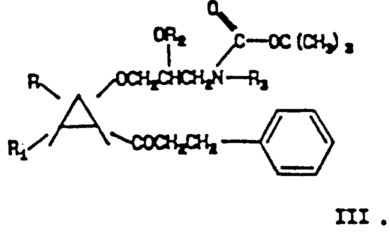 durch Behandlung mit Säure in die entsprechenden Verbindungen der Formel I überführt, in denen R₄ Wasserstoff bedeutet, und die Verbindungen erwünschtenfalls in ein Säureadditionssalz überführt.

2. Verfahren nach Anspruch 1,
**dadurch gekennzeichnet,**
daß Alkyl, Alkenyl, Alkinyl, Alkoxy und Acyl 1 bis 4 C-Atome aufweisen.

3. Verfahren nach Anspruch 1 oder 2,
**dadurch gekennzeichnet,**
daß R, R₁ = H und/oder CH₃ bedeuten.

4. Verfahren nach Anspruch 3,
**dadurch gekennzeichnet**,
daß A den Thiophenring, R Wasserstoff und R₁ Methyl bedeuten.

5. Verfahren nach Anspruch 3,
**dadurch gekennzeichnet,**
daß A den Benzolring und R und R₁ Wasserstoff bedeuten.

6. Verfahren nach den Ansprüchen 2 bis 5,
**dadurch gekennzeichnet,**
daß R₃ Isobutyl bedeutet.

7. Verfahren nach Anspruch 1,
**dadurch gekennzeichnet**,
daß man 1-[3-(2-Methoxy-3-(2-methylpropylamino)-propoxy)-4-methyl-2-thienyl]-3-phenyl-1-propanon herstellt.

8. Verfahren nach Anspruch 1,
**dadurch gekennzeichnet,**
daß man 1-[3-(2-Methoxy-3-(2-methylpropylamino)-propoxy)-4-methyl-2-thienyl]-3-phenyl-1-propanon, Hydrochlorid herstellt.

9. Verfahren nach Anspruch 1,
**dadurch gekennzeichnet**,
daß man 2-(2-Methoxy-3-propylamino-propoxy)-3-phenyl-propiophenon herstellt.

10. Verfahren nach Anspruch 1,
**dadurch gekennzeichnet**,
daß man 2-(2-Methoxy-3-propylamino-propoxy)-3-phenyl-propiophenon, Hydrochlorid herstellt.

## Claims (Claims for the following Contracting State(s): AT, BE, CH, DE, DK, FR, GB, IT, LI, LU, NL, SE)

1. Aryloxy-alkylamine derivatives of the general formula I 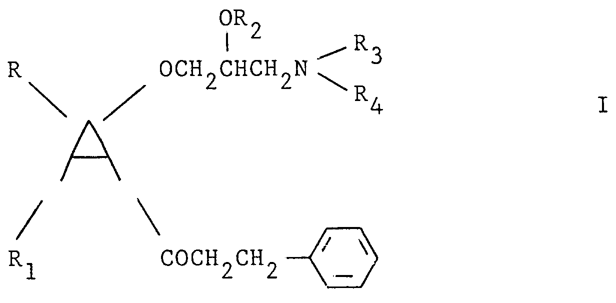 in which A signifies a benzene or thiophene ring, R and R₁, independently of one another, each hydrogen, alkyl, halogen, CF₃ or alkoxy, R₂ alkyl, cycloalkyl, alkenyl, alkynyl, alkaryl or saturated or unsaturated aliphatic or aromatic acyl, R₃ and R₄ each hydrogen, alkyl, alkenyl, alkynyl or cycloalkyl with, in each case, up to 8 C-atoms, whereby R₃ and R₄can be the same or different but are not simultaneously hydrogen or R₃ and R₄, together with the nitrogen atom connecting them, form a 5 to 7-membered saturated ring or a saturated heterocyclic ring which can possibly contain an oxygen or nitrogen atom as a further heteroatom in the ring, whereby an additional nitrogen atom can be substituted by an alkyl radical with up to 3 C-atoms, and their acid-addition salts.

2. Derivative according to claim 1, characterised in that the alkyl, alkenyl, alkynyl, alkoxy and acyl have 1 to 3 C-atoms.

3. Derivative according to claim 1 or 2, characterised in that R, R₁ = H and/or CH₃.

4. Derivative according to claim 3, characterised in that A signifies the thiophene ring, R hydrogen and R₁ methyl.

5. Derivative according to claim 3, characterised in that A signifies the benzene ring and R and R₁ hydrogen.

6. Derivative according to claims 2 to 5, characterised in that R₃ signifies isobutyl.

7. 1-[3-(2-Methoxy-3-(2-methylpropylamino)-propoxy)-4-methyl-2-thienyl]-3-phenyl-1-propanone.

8. 1-[3-(2-Methoxy-3-(2-methylpropylamino)-propoxy)-4-methyl-2-thienyl]-3-phenyl-1-propanone hydrochloride.

9. 2-(2-Methoxy-3-propylaminopropoxy)-3-phenylpropiophenone.

10. 2-(2-Methoxy-3-propylaminopropoxy)-3-phenylpropiophenone hydrochloride.

11. Process for the preparation of a derivative according to one of claims 1 to 10, characterised in that one reacts a compound of the general formula II or IIa, respectively 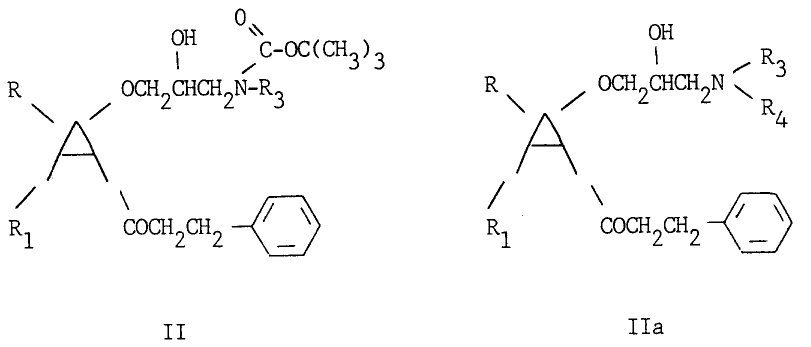 in which A, R, R₁, R₃ and R₄ have the meaning given for formula I, with an alkyl transmitter, such as an alkyl halide, alkyl sulphuric acid ester or alkyl sulphonic acid ester, or with an acyl halide in the presence of at least one equivalent of a base in an inert organic solvent, possibly converts the compounds obtained of the general formula III 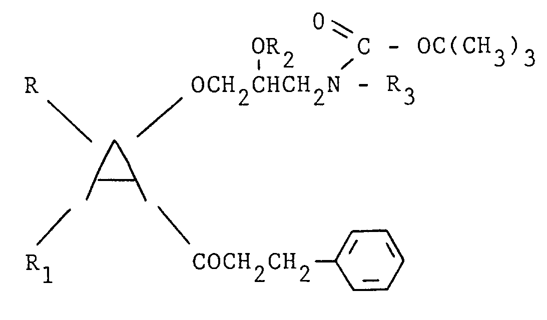 by treatment with acid into the corresponding compounds of the formula I, in which R₄ signifies hydrogen, and, if desired, converts the compounds into an acid-addition salt.

12. Medicaments, characterised by a content of at least one compound of the general formula I or of a pharmacologically acceptable salt thereof in combination with usual galenical adjuvant and/or carrier materials.

13. Medicaments according to claim 12 for the treatment of diseases of the heart-circulatory system, especially anti-arrhythmia.

## Claims (Claims for the following Contracting State(s): ES)

1. Process for the preparation of aryloxy-alkylamine derivatives of the general formula I 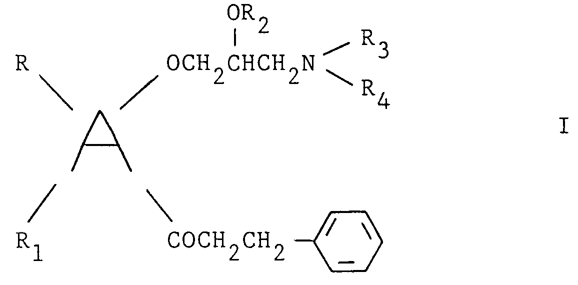 in which A signifies a benzene or thiophene ring, R and R₁, independently of one another, each hydrogen, alkyl, halogen, CF₃ or alkoxy, R₂ alkyl, cycloalkyl, alkenyl, alkynyl, alkaryl or saturated or unsaturated aliphatic or aromatic acyl, R₃ and R₄ each hydrogen, alkyl, alkenyl, alkynyl or cycloalkyl with, in each case, up to 8 C-atoms, whereby R₃ and R₄ can be the same or different but are not simultaneously hydrogen or R₃ and R₄, together with the nitrogen atom connecting them, form a 5 to 7-membered saturated ring or a saturated heterocyclic ring which can possibly contain an oxygen or nitrogen atom as a further heteroatom in the ring, whereby an additional nitrogen atom can be substituted by an alkyl radical with up to 3 C-atoms, and of their acid-addition salts, characterised in that one reacts a compound of the general formula II or IIa, respectively 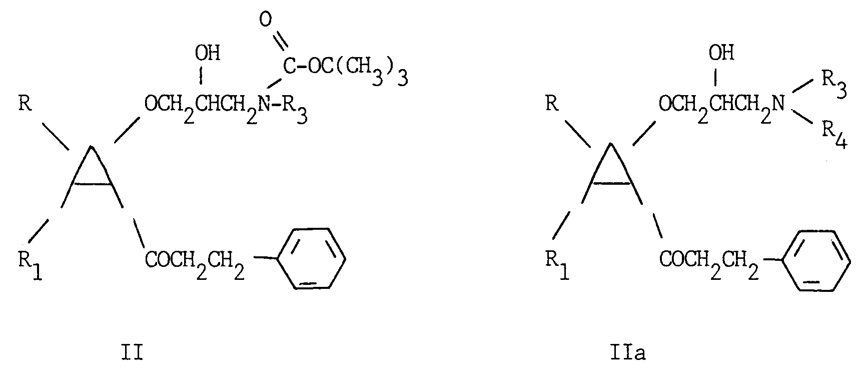 in which A, R, R₁, R₃ and R₄ have the meaning given for formula I, with an alkyl transmitter, such as an alkyl halide, alkyl sulphuric acid ester or alkyl sulphonic acid ester, or with an acyl halide in the presence of at least one equivalent of a base in an inert organic solvent, possibly converts the compounds obtained of the general formula III 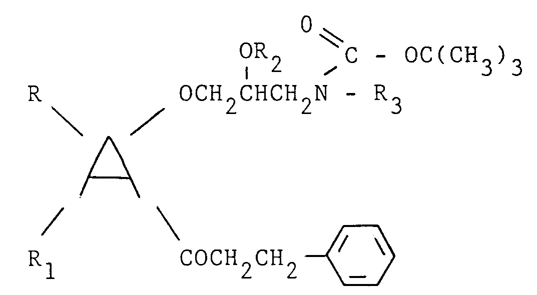 by treatment with acid into the corresponding compounds of the formula I, in which R₄ signifies hydrogen, and, if desired, converts the compounds into an acid-addition salt.

2. Process according to claim 1, characterised in that alkyl, alkenyl, alkynyl, alkoxy and acyl have 1 to 4 C-atoms.

3. Process according to claim 1 or 2, characterised in that R, R₁ = H and/or CH₃.

4. Process according to claim 3, characterised in that A signifies the thiophene ring, R hydrogen and R₁ methyl.

5. Process according to claim 3, characterised in that A signifies the benzene ring and R and R₁ hydrogen.

6. Process according to claims 2 to 5, characterised in that R₃ signifies isobutyl.

7. Process according to claim 1, characterised in that one prepares 1-[3-(2-methoxy-3-(2-methylpropylamino)-propoxy)-4-methyl-2-thienyl]-3-phenyl-1-propanone.

8. Process according to claim 1, characterised in that one prepares 1-[3-(2-methoxy-3-(2-methylpropylamino)-propoxy]-4-methyl-2-thienyl]-3-phenyl-1-propanone hydrochloride.

9. Process according to claim 1, characterised in that one prepares 2-(2-methoxy-3-propylaminopropoxy)-3-phenylpropiophenone.

10. Process according to claim 1, characterised in that one prepares 2-(2-methoxy-3-propylaminopropoxy)-3-phenylpropiophenone hydrochloride.

## Revendications (Revendications pour l'(les) Etat(s) contractant(s) suivant(s): AT, BE, CH, DE, DK, FR, GB, IT, LI, LU, NL, SE)

1. Dérivés d'aryloxyalkylamines de formule générale I 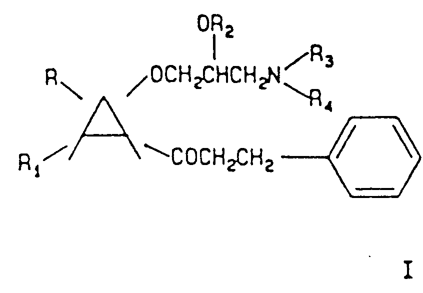 dans laquelle A représente un noyau benzène ou thiophène,
R et R₁ représentent chacun, indépendamment l'un de l'autre, un hydrogène, un alkyle, un halogène, CF₃ ou un alcoxy,
R₂ représente un alkyle, un cycloalkyle, un alcényle, un alcynyle, un alkaryle, ou un acyle aliphatique saturé ou insaturé ou aromatique,
R₃ et R₄ représentent chacun un hydrogène, un alkyle, un alcényle, un alcynyle ou un cycloalkyle ayant chacun jusqu'à 8 atomes de carbone, R₃ et R₄ pouvant être identiques ou différents mais n'étant pas en même temps un atome d'hydrogène, ou bien R₃ et R₄ forment ensemble, avec l'atome d'azote auquel ils sont liés, un noyau saturé de 5 à 7 chaînons ou un noyau hétérocyclique saturé qui peut éventuellement contenir un atome d'oxygène ou d'azote comme autre hétéroatome cyclique, l'atome d'azote supplémentaire pouvant être substitué par un reste alkyle d'au plus 3 atomes de carbone, et leurs sels d'addition d'acides.

2. Dérivé selon la revendication 1, caractérisé en ce que les restes alkyle, alcényle, alcynyle, alcoxy et acyle comportent 1 à 4 atomes de carbone.

3. Dérivé selon la revendication 1 ou 2, caractérisé en ce que R, R₁ représentent H et/ou CH₃.

4. Dérivé selon la revendication 3, caractérisé en ce que A représente le noyau thiophène, R est un hydrogène et R₁ représente un méthyle.

5. Dérivé selon la revendication 3, caractérisé en ce que A représente le noyau phényle et R et R₁ représentent l'hydrogène.

6. Dérivé selon les revendications 2 à 5, caractérisé en ce que R₃ représente un isobutyle.

7. 1-[3-(2-méthoxy-3-(2-méthylpropylamino)propoxy)-4-méthyl-2-thiényl]-3-phényl-1-propanone.

8. Chlorhydrate de la 1-[3-(2-méthoxy-3-(2-méthylpropylamino)-propoxy)-4-méthyl-2-thiényl]-3-phényl-1-propanone.

9. 2-(2-méthoxy-3-propylaminopropoxy)-3-phénylpropiophénone.

10. Chlorhydrate de la 2-(2-méthoxy-3-propylaminopropoxy)-3-phénylpropiophénone.

11. Procédé de préparation d'un dérivé selon l'une des revendications 1 à 10, caractérisé en ce que l'on fait réagir un composé de formule générale II ou IIa 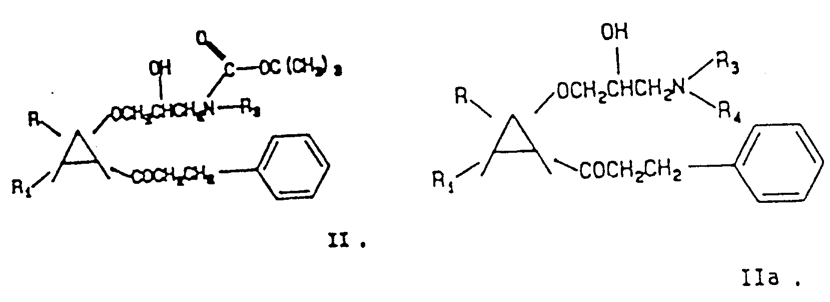 dans lesquelles A, R, R₁, R₃ et R₄ ont la signification indiquée pour la formule I, avec un agent d'alkylation tel qu'un halogénure d'alkyle, un sulfate d'alkyle ou un sulfonate d'alkyle, ou avec un halogénure d'acyle, en présence d'au moins un équivalent d'une base dans un solvant organique inerte, on transforme éventuellement, par traitement avec un acide, le composé de formule générale III obtenu 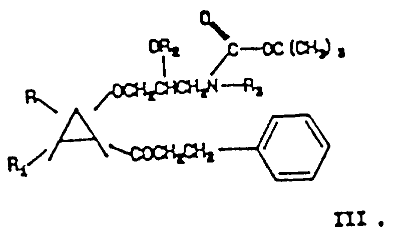 en le composé correspondant de formule I dans lequel R₄ représente un hydrogène, et, si désiré, on transforme le composé en un sel d'addition d'acide.

12. Médicament caractérisé en ce qu'il contient au moins un composé de formule générale I ou un de ses sels pharmacologiquement acceptables, en combinaison avec des produits auxiliaires et/ou supports galéniques classiques.

13. Médicament selon la revendication 12 pour le traitement de maladies du système cardiovasculaire, en particulier comme antiarythmique.

## Revendications (Revendications pour l'(les) Etat(s) contractant(s) suivant(s): ES)

1. Procédé de préparation de dérivés d'aryloxyalkylamines de formule générale I 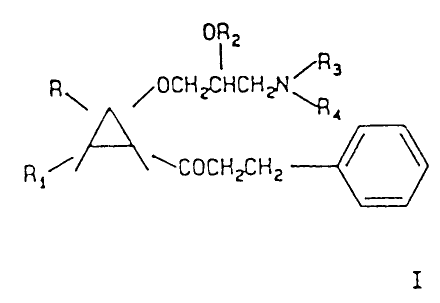 dans laquelle A représente un noyau benzène ou thiophène,
R et R₁ représentent chacun, indépendamment l'un de l'autre, un hydrogène, un alkyle, un halogène, CF₃ ou un alcoxy,
R₂ représente un alkyle, un cycloalkyle, un alcényle, un alcynyle, un alkaryle, ou un acyle aliphatique saturé ou insaturé ou aromatique,
R₃ et R₄ représentent chacun un hydrogène, un alkyle, un alcényle, un alcynyle ou un cycloalkyle ayant chacun jusqu'à 8 atomes de carbone, R₃ et R₄ pouvant être identiques ou différents mais n'étant pas en même temps un atome d'hydrogène, ou bien R₃ et R₄ forment ensemble, avec l'atome d'azote auquel ils sont liés, un noyau saturé de 5 à 7 chaînons ou un noyau hétérocyclique saturé qui peut éventuellement contenir un atome d'oxygène ou d'azote comme autre hétéroatome cyclique, l'atome d'azote supplémentaire pouvant être substitué par un reste alkyle d'au plus 3 atomes de carbone, et de leurs sels d'addition d'acides, caractérisé en ce que l'on fait réagir un composé de formule générale II ou IIa 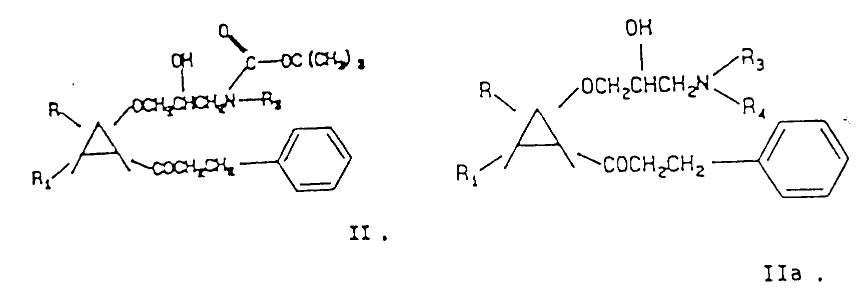 dans lesquelles A, R, R₁, R₃ et R₄ ont la signification indiquée pour la formule I, avec un agent d'alkylation tel qu'un halogénure d'alkyle, un sulfate d'alkyle ou un sulfonate d'alkyle, ou avec un halogénure d'acyle, en présence d'au moins un équivalent d'une base dans un solvant organique inerte, on transforme éventuellement, par traitement avec un acide, le composé de formule générale III obtenu 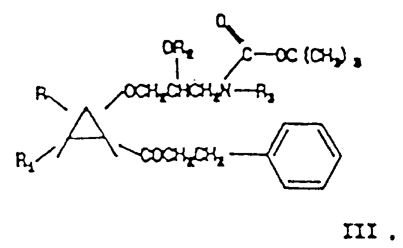 en le composé correspondant de formule I dans lequel R₄ représente un hydrogène, et, si désiré, on transforme le composé en un sel d'addition d'acide.

2. Procédé selon la revendication 1, caractérisé en ce que les restes alkyle, alcényle, alcynyle, alcoxy et acyle comportent 1 à 4 atomes de carbone.

3. Procédé selon la revendication 1 ou 2, caractérisé en ce que R, R₁ représentent H et/ou CH₃.

4. Procédé selon la revendication 3, caractérisé en ce que A représente le noyau thiophène, R est un hydrogène et R₁ représente un méthyle.

5. Procédé selon la revendication 3, caractérisé en ce que A représente le noyau phényle et R et R₁ représentent l'hydrogène.

6. Procédé selon les revendications 2 à 5, caractérisé en ce que R₃ représente un isobutyle.

7. Procédé selon la revendication 1, caractérisé en ce que l'on prépare la 1-[3-(2-méthoxy-3-(2-méthylpropylamino)propoxy)-4-méthyl-2-thiényl]-3-phényl-1-propanone.

8. Procédé selon la revendication 1, caractérisé en ce que l'on prépare le chlorhydrate de la 1-[3-(2-méthoxy-3-(2-méthylpropylamino)propoxy)-4-méthyl-2-thiényl]-3-phényl-1-propanone.

9. Procédé selon la revendication 1, caractérisé en ce que l'on prépare la 2-(2-méthoxy-3-propylaminopropoxy)-3-phénylpropiophénone.

10. Procédé selon la revendication 1, caractérisé en ce que l'on prépare le chlorhydrate de la 2-(2-méthoxy-3-propylaminopropoxy)-3-phénylpropiophénone.
